# EUROPÄISCHE PATENTANMELDUNG

(11) **EP 0 953 575 A1**
(43) Veröffentlichungstag der Anmeldung: **03.11.1999**
(21) Anmeldenummer: 98107911.4
(22) Anmeldetag: 30.04.1998
(51) Int. Cl.: C07K 14/47, C07K 19/00

(54) **Aktives Hedgehog-Protein-Konjugat, Verfahren zur Herstellung und Verwendung**

(71) Anmelder: Roche Diagnostics GmbH, 68305 Mannheim (DE)
(72) Erfinder: Die Erfindernennung liegt noch nicht vor

(57) **Zusammenfassung**

Ein Hedgehog-Konjugat, dadurch gekennzeichnet, daß es kovalent gebunden enthält: a) ein Polypeptid bestehend aus 10 bis 30 hydrophoben Aminosäuren und/oder Aminosäuren, welche Transmembranhelices bilden und positiv geladen sind, b) 1 bis 4 aliphatische, gesättigte oder ungesättigte Kohlenwasserstoffreste mit einer Kettenlänge von 10 bis 24 C-Atomen und mit einer hydrophoben Wirkung, oder c) eine hydrophobe Thioverbindung, zeigt eine um ein Vielfaches erhöhte Aktivität und ist als pharmazeutisches Mittel geeignet.

## Beschreibung

Gegenstand der Erfindung ist ein Hedgehog-Protein Konjugat mit erhöhter Aktivität, Verfahren zu dessen Herstellung und dessen therapeutische Verwendung.

Unter Hedgehog (hh)-Proteinen versteht man eine Familie von sekretierten Signalproteinen, die verantwortlich sind für die Ausbildung einer Vielzahl von Strukturen in der Embryogenese (J.C. Smith, Cell 76 (1994) 193 - 196, N. Perrimon, Cell 80 (1995) 517 - 520, C. Chiang et al., Nature 83 (1996) 407, M.J. Bitgood et al., Curr. Biol. 6 (1996) 296, A. Vortkamp et al., Science 273 (1996) 613, C.J. Lai et al., Development 121 (1995) 2349). Bei der Biosynthese wird nach Abspaltung der Signalsequenz und autokatalytischer Spaltung eine 20 kD N-terminale Domäne und eine 25 kD C-terminale Domäne erhalten. Im natürlich vorkommenden Protein wird die N-terminale Domäne nach Abspaltung der C-terminalen Domäne an ihrem C-Terminus Cholesterin-modifiziert (J.A. Porter et al., Science 274 (1996) 255 - 259. In höheren Lebewesen besteht die hh-Familie zumindest aus drei Mitgliedern, nämlich Sonic, Indian und Desert hh (Shh, Ihh, Dhh; M. Fietz et al., Development (Suppl.) (1994) 43 - 51). Für Hedgehog-Proteine, welche rekombinant hergestellt wurden, wurde eine unterschiedliche Aktivität nach Herstellung in Prokaryonten und Eukaryonten beobachtet (M. Hynes et al., Neuron 15 (1995) 35 - 44 und T. Nakamura et al., Biochem. Biophys. Res. Comm. 237 (1997) 465 - 469).

Hynes et al. vergleichen die Aktivität von hh im Überstand von transformierten "human embryonic kidney 293 cells" (eukaryontischer hh) mit aus E.coli hergestellten hh und finden eine vierfach höhere Aktivität des hh aus den Überständen der Nierenzellinie. Als Ursache dieser erhöhten Aktivität wird ein potentieller zusätzlicher "accessory factor", der nur in eukaryontischen Zellen exprimiert wird, eine posttranslationale Modifikation, ein unterschiedlicher N-Terminus, da der aus E.coli isolierte hh 50 % einer hh-Form enthält, welche zwei zusätzliche N-terminale Aminosäuren (Gly-Ser) trägt, oder um 5 - 6 Aminosäuren verkürzt ist, oder ein höherer Aggregatzustand (z. B. durch Bindung an Nickel-Agarose beads) von hh diskutiert.

Nakamura et al. vergleichen die Aktivität von shh im Überstand von transformierten "chicken embryo fibroblasts" mit einem aus E.coli isolierten shh Fusionsprotein, das noch einen N-terminalen Polyhistidinteil aufweist. Das shh im Überstand der Fibroblasten hat bezüglich der Stimulation von alkalischer Phosphatase (AP) in C3H10T ½ Zellen eine siebenfach höhere Aktivität als das gereinigte E.coli Protein. Als Ursache der erhöhten Aktivität wird ein Synergismus von hh mit Molekülen, wie beispielsweise bone morphogenic proteins (BMPs) diskutiert, die nur im Überstand von eukaryontischen Zellen vorhanden sind und in Kombination mit hh die stärkere Induktion der AP verursacht.

Von Kinto et al., FEBS Letters, 404 (1997) 319 - 323 wurde beschrieben, daß hh sekretierende Fibroblasten bei i.m. Implantation auf Kollagen eine ektopische Knochenbildung induzieren. Eine derartige Aktivität ist für ein isoliertes hh Protein jedoch nicht bekannt.

Aufgabe der Erfindung ist es, hh-Proteine (Polypeptide) zur Verfügung zu stellen, welche eine deutlich verbesserte Aktivität gegenüber den bekannten Formen zeigen.

Die Aufgabe wird gelöst durch ein nach rekombinanter Herstellung artifiziell lipophilisiertes Hedgehog-Protein. Eine solche Lipophilisierung erfolgt vorzugsweise durch chemische Modifikation. Vorzugsweise enthält ein solches Hedgehog-Konjugat (vorzugsweise am C- und/oder N-Terminus) kovalent gebunden ein zusätzliches Polypeptid, bestehend aus 10 - 30 vorzugsweise hydrophoben Aminosäuren und/oder solchen Aminosäuren, welche Transmembranhelices bilden. Besonders bevorzugt enthält das zusätzliche Polypeptid 2 - 12 Lysine und/oder Arginine, jedoch keinen Polyhistidinanteil, der zur Reinigung des Konjugats an einer Ni-Chelatsäule geeignet ist. Ebenfalls bevorzugt ist es, (vorzugsweise am C- und/oder N-Terminus) kovalent 1 - 4 aliphatische, gesättigte oder ungesättigte Kohlenwasserstoffreste mit einer Kettenlänge von 10 - 24 C-Atomen und mit einer lipophilen (hydrophoben) Wirkung zu binden. Weiterhin ist es bevorzugt, hydrophobe Thioverbindungen, wie insbesondere Thiocholesterol, über eine oxidativ gebildete Disulfidbrücke (vorzugsweise an den C- und/oder N-Terminus und dort an das N-terminale Cystein) von hh-Proteinen kovalent zu koppeln.

Durch solche lipophilisierenden Reste wird das Protein hydrophobisiert und damit seine Interaktion mit Lipidmembranen von eukaryontischen Zellen, insbesondere von Säugerzellen, verbessert.

Unter einem lipophilisierten Protein, gemäß der Erfindung, ist demnach ein hydrophobisiertes Protein zu verstehen, welches gegenüber einem unmodifizierten Protein eine erhöhte Oberflächenhydrophobizität zeigt, wodurch seine Affinität für apolare Moleküle oder Amphiphile erhöht wird. Die Zunahme des Hypophilisierungsgrades eines Proteins kann durch den Grad der Integration in eine Lipidschicht gemessen werden, wie beispielsweise beschrieben von Haque, Z. et al., J. Agric. Food Chem. 30 (1982) 481. Verfahren zur hydrophoben (lipophilisierenden) Modifikation von Proteinen sind beispielsweise beschrieben von Haque, Z. et al., J. Agric. Food Chem. 31 (1983) 1225 - 1230; Webb, R.J. et al., Biochemistry 37 (1998) 673 - 679; Hancock, J.F., Cell 63 (1990) 133 - 139, A Practical guide to membrane protein purification, Ed. G. v. Jagow, Hermann Schägger (1994) (Kapitel 16, Seiten 535 - 554).

Es hat sich überraschenderweise gezeigt, daß derartig lipophilisierte Hedgehog-Proteine (im weiteren auch als Hedgehog-Konjugate (hh-Konjugate) bezeichnet), insbesondere in einer pharmazeutischen Formulierung und in vitro, eine drastisch erhöhte Aktivität, vorzugsweise mindestens 10fach, besonders bevorzugt 10³ - 10⁵-fach gegenüber nicht-modifizierten Hedgehog-Proteinen (z. B. nach cytoplasmatischer Expression in E.coli) zeigen. Besonders überraschend ist zusätzlich, daß solche erfindungsgemäßen Hedgehog-Konjugate besonders vorteilhalt für eine lokale Therapie, vorzugsweise am Knochen, am Knorpel, an Nervenzellen (bei Nervenläsionen oder neurodegenerativen Erkrankungen) oder in Muskelgewebe angewendet werden können.

Aus Yang et al., Development 124 (1997) 4393-4404, ist bekannt, daß für eine pharmazeutisch wirksame in vivo Aktivität am Wirkort im Körper hohe lokale Hedgehog-Konzentrationen über einen Zeitraum von mindestens 16 h vorherrschen müssen. Das von Yang et al. hierzu beschriebene Trägersystem wie das Hedgehog-beladene Chromatographie-Medium Affigel CM, die von Marti et al. in Nature 375 (1995) 322-325 beschriebene Ni-Agarose oder das von Lopez-Martinez et al. in Curr. Biol. 5 (1995) 791-796 verwendete Affigel Blue oder die darin verwendeten Heparin-Agarose-Partikel sind für eine pharmazeutische Anwendung wenig geeignet, da sie immunogen sind und entzündliche Reaktionen hervorrufen können.

Die erfindungsgemäßen Konjugate dienen als neue Wirkstoffe für die Herstellung von pharmazeutischen Darreichungsformen. Insgesamt resultiert aus der Kopplung ein verbessertes pharmakokinetisches Profil des Hedgehog-Proteins. Durch den hydrophoben Kohlenwasserstoffrest erfolgt eine Lokalisierung des Hedgehog-Proteins an die Membran von Zielzellen, wodurch neben einer erleichterten Integration in das Zellinnere vor allem eine für die optimale pharmakologische Wirkung wesentliche langanhaltende Zelloberflächenständigkeit erreicht wird.

Für die erfindungsgemäßen Konjugate ist eine zusätzliche Kopplung an einen Träger zur langsamen Freisetzung nicht unbedingt erforderlich. Die erfindungsgemäßen Hedgehog-Konjugate sind auch ohne daß eine verzögerte Freisetzung von einem Träger über einen langen Zeitraum (mehrere Tage) erfolgt, am Wirkort im Körper hochaktiv. Zweckmäßig wird zur lokalen Applikation der erfindungsgemäßen Hedgehog-Konjugate dennoch eine pharmazeutische Zusammensetzung verwendet, welche das erfindungsgemäße Konjugat mit einer Trägermatrix zusammen enthält. Die Trägermatrix dient im Wesentlichen zur Erleichterung der lokalen Applikation, insbesondere dadurch, daß eine solche pharmazeutische Zusammensetzung eine für die lokale Applikation geeignete Mindestviskosität besitzt. Vorzugsweise ist die pharmazeutische Zusammensetzung im pH-Bereich zwischen pH 4 und 9 gepuffert und enthält ein oder mehrere nicht-ionische Detergentien wie z.B. Polyoxysorbat (z.B. Tween® 20, Tween® 80), Triton® X-100, oder ionische Detergenzien wie Natriumdeoxycholat, Natriumcholat, Natriumtaurodeoxycholat.

In einer bevorzugten Ausführungsform wird ein hh-Protein exprimiert, das am N- und/oder C-Terminus zusätzlich 10 - 30 überwiegend hydrophobe Aminosäuren enthält, da diese ebenfalls in die Membran von Zellen inkorperiert werden [Webb et al., Biochemistry 37 (1998) 673 - 679, Skolnick et al., Biol. Membranes (1996) 536 - 554; ed.: Merz and Roux]. Unter hydrophoben Aminosäuren im Sinne der Erfindung sind Aminosäuren zu verstehen, die eine negative freie Energie beim Übergang von der wäßrigen Phase in eine hydrophobe/organische Phase aufweisen. Weiterhin sind auch N- und/oder C-terminale zusätzliche Sequenzen zu einer Erhöhung der Aktivität von hh-Proteinen geeignet, die bekannterweise Transmembranhelices ausbilden, wie z.B. das M28-Peptid, oder die als Helix mit der Oberfläche von Membranen interagieren, wie z.B. Maginin 2 (Skolnick et al., 1996).

In einer weiteren bevorzugten Ausführungsform ist der N- und/oder C-Terminus des Hedgehog-Proteins durch einen Polypeptidrest, welcher 2 - 12 Lysine und/oder Arginine enthält, modifiziert. In diesem Fall kann auf die Modifikation mit dem Kohlenwasserstoffrest verzichtet werden.

Unter einem aliphatischen, gesättigten oder ungesättigten Kohlenwasserstoffrest mit hydrophober Wirkung und einer Kettenlänge von 10 - 24, vorzugsweise 12 - 18 C-Atomen, ist vorzugsweise ein gesättigter oder ein- bis mehrfach ungesättigter Fettsäure- oder Alkylalkoholrest zu verstehen, gegebenenfalls unterbrochen durch ein Sauerstoff- oder Schwefelatom oder eine Carbonylgruppe. Besonders bevorzugte gesättigte Fettsäuren sind: Laurinsäure, Mynstinsäure, Palmitinsäure, Stearinsäure, Arachinsäure und Behensäure. Bevorzugte einfach ungesättigte Fettsäuren sind Palmitoleinsäure und Ölsäure. Besonders bevorzugte mehrfach ungesättigte Fettsäuren sind Linolsäure, Linolensäure und Arachidonsäure. Solche Fettsäurereste sind vorzugsweise über eine Ester-, Säureamid- oder Thioesterbindung an reaktive Gruppen des Proteins gekoppelt.

Die Anzahl der hydrophoben Kohlenwasserstoffketten pro Proteinmolekül kann durch die Reaktionsbedingungen (z.B. Verdünnung) oder durch die Auswahl der zu modifizierenden Aminosäure zweckmäßig gesteuert werden. Beispielsweise enthält shh drei Cysteine, von denen das N-terminale Cystein besonders reaktiv ist. In diesem Fall kann durch die Reaktionsführung entweder das N-terminale Cystein mit der oder den hydrophoben Kohlenwasserstoffketten modifiziert werden. Ebenfalls möglich ist es, statistisch zwei oder nahezu alle drei Cysteine zu modifizieren. Bei der Modifikation anderer Aminosäuren ist es zwar bevorzugt, konkret definierte Aminosäure zu modifizieren, für die pharmazeutische Zusammensetzung ist es jedoch auch möglich, solche derivatisierten Hedgehog-Proteine zu verwenden, bei denen eine statistische Verteilung der Kohlenwasserstoffketten-Modifikation von ca. 1 bis ca. 3 Ketten pro Molekül vorliegt. Eine höhere Anzahl von Kohlenwasserstoffketten pro Molekül ist zwar durchaus geeignet, jedoch nimmt dadurch die Löslichkeit in einer pharmazeutischen Zusammensetzung ab und es können sich Störungen der aktiven dreidimensionalen Proteinstruktur ergeben. Bei einer Kopplung mit langkettigen Alkylgruppen (C16 - C24) werden bevorzugt nur 1 - 2 Kohlenstoffketten angeknüpft, bei einer Kopplung von kurzkettigen Kohlenstoffketten jedoch bevorzugt 2 - 3 Alkylgruppen angekoppelt.

In einer bevorzugten Ausführungsform kann die Derivatisierung auch darin bestehen, daß zwei hydrophobe Kohlenwasserstoffketten an eine Aminosäure gekoppelt sind. Dies kann beispielsweise durch die Kopplung eines Fettsäurediglycerids an die Aminosäure erreicht werden.

Die Kohlenwasserstoffkette oder die Kohlenwasserstoffketten werden zweckmäßig an reaktive Gruppen des Proteins, beispielsweise an freie Hydroxy-, Mercapto-, Carboxy- oder Aminogruppen über eine Amidbindung, eine Ester- oder Thioesterbindung gekoppelt. Derartige Verfahren sind dem Fachmann bekannt und beispielsweise beschrieben in Wong, S.S., Chemistry of Protein Conjugation and Cross Linking, CRC Press, Boca Raton, FL., USA, 1993. Beispielsweise kann die Kopplung von Fettsäuren als Thioester mit Coenzym A erfolgen (z.B. Palmitoyl-Coenzym A), über eine Succinimidester- oder N-Maleimid-Kopplung (z.B. Palmitinsäure-N-Hydroxy-Succinimidester), über ein Fettsäureanhydrid, Fettsäureimidazolid oder Säurechlorid.

Für Palmitoyl-CoA, Stearoyl-CoA oder Myristeyl-CoA sind Kopplungsverfahren beispielsweise beschrieben von Ross et al., J. Neurosci. Res. 21 (1988) 35 - 44, Bizzozero et al., J. Biol. Chem. 262 (1987) 2138 - 2145, oder für Tubulin von Ozols, J. et al., Molec. Biol. of the Cell 8 (1997) 637 - 645. Eine Derivatisierung mit Fettsäureanhydriden wurde z.B. für Ovalbumin (Segawa, A. et al., Int. Archs Allergy appl. Immun. 66 (1981) 189 - 199) oder Peptide (Yadav, S.P. et al., Biochem. Biophys. Res. Comm. 205 (1994) 1688 - 1695) beschrieben. Auch für eine Acylierung mit Fettsäuresuccinimidestern existieren zahlreiche Beispiele wie z.B. für Casein (Haque, Z. et al., J. Agric. Food Chem. 31 (1983) 1225 - 1230), (Haque, Z. et al., Agric. Biol. Chem. 46 (1982) 597 - 599). Ebenso kann über eine Aldehydgruppe am Fusionspartner (z.B. Palmitoyl-Cys-CHO) N-terminal an Cystein gebunden werden (Liu et al., Proc. Natl. Acad. Sci. USA 91 (1994) 6584-6588). Eine N-terminale Kopplung an Serin kann durch Umwandlung in eine Aldehydgruppe, Reaktion mit Hydrazid (z.B. Palmitoyl-Cys-Hydrazid) und Stabilisierung des entstandenen Hydrazons (z.B. durch Reduktion mit NaBH₃CN) erfolgen (Gaertner et al., Bioconjugate Chem. 3 (1992) 262-268).

Die hydrophobe Kohlenwasserstoffkette wird, abhängig von der Kopplungschemie, beispielsweise in Form eines Ethers, Thioethers, Esters, Thioesters oder Amids an die Seitengruppe der reaktiven Aminosäuren Serin, Threonin, Glutaminsäure, Asparginsäure, Cystein, Arginin oder Lysin gebunden. Methoden zur spezifischen Kopplung an bestimmte Aminosäuren sind von Wong, S.S., in Chemistry of Protein Conjugation and Cross Linking, CRC Press Inc., Boca Raton, FL, USA (1993) und Lundblad in Techniques in Protein Modification (1995) beschrieben.

In einer weiteren bevorzugten Ausführungsform wird Thiocholesterol über eine oxidativ gebildete Disulfidbrücke in Gegenwart von solubilisierenden Detergenzien wie insbesondere Na-deoxycholat, -cholat, -taurodeoxycholat oder Triton® X-100 an die Thiol-Gruppe insbesondere des N-terminalen Cysteins gekoppelt. Im Gegensatz zum natürlicherweise C-terminal mit Cholesterol modifizierten N-terminalen hh-Fragment entsteht dadurch eine hh-Form, die N-terminal ein Thiocholesterol enthält. Diese Form weist eine ähnlich erhöhte Aktivität wie die natürliche Form auf, ist jedoch wesentlich einfacher und in größeren Mengen herstellbar. Aufgrund der cytoplasmatischen Labilität von Disulfidbrücken hat diese hh-Form kein oder nur geringes immunogenes Potential.

Zur Erhöhung der Löslichkeit von lipophil modifizierten hh-Proteinen ist es weiterhin bevorzugt, die Derivatisierung und/oder anschließende Reinigung bzw. pharmazeutische Zubereitung in Gegenwart von löslichen, anionischen Polysacchariden wie Suramin, Heparin durchzuführen.

Unter Aktivität im Sinne der Erfindung ist die Aktivität an alkalischer Phosphatase zu verstehen, die das Polypeptid in Säugerzellen induzieren kann (Aktivität im alkalischen Phosphatasetest). Dabei wird eine Maus-Fibroblasten-Zellinie in einem Medium, welches fötales Kälberserum enthält kultiviert. Anschließend wird sterilfiltrierte Probe zugesetzt, nach ca. 5 Tagen die Zellen aufgeschlossen und im Zellysat die alkalische Phosphatase z.B. über die Spaltung eines chromogenen Substrats (pNP, p-Nitrophenol) bestimmt (J. Asahina, Exp. Cell. Res. 222 (1996) 38 - 47 und T. Nakamura (1997)).

Unter einem Hedgehog-Protein, gemäß der Erfindung, ist ein sekretiertes Signalprotein zu verstehen, welches für die Ausbildung einer Vielzahl von Strukturen in der Embryogenese verantwortlich ist. Besonders bevorzugt verwendet wird Sonic, Indian- oder Desert hh (Fietz, M., et al., Development (Suppl.) (1994) 43-51). Bevorzugt wird ein hh-Protein einer Sequenz, wie sie in der EMBL-Datenbank unter Nr. L38518 beschrieben ist, verwendet. Proteine der Hedgehog-Familie zeigen eine ausgeprägte Homologie in der Aminosäuresequenz, weshalb es ebenso bevorzugt ist, solche Nukleinsäuren zu exprimieren, welche für Hedgehog-Proteine codieren, die zu 80% oder mehr homolog mit der oben genannten Sequenz von Sonic Hedgehog-Protein (shh) sind.

Das humane Sonic Hedgehog-Precursorprotein besteht aus den Aminosäuren 1 - 462 der in der EMBL-Datenbank unter Nr. L38518 beschriebenen Sequenz. Die Aminosäuren 1 - 23 stellen dabei das Signalpeptid dar, die Aminosäuren 24 - 197 die mature Signaldomäne, die Aminosäuren 32 - 197 die um acht Aminosäuren verkürzte Signaldomäne und die Aminosäuren 198 - 462 die autoprozessierende C-terminale Domäne nach autoproteolytischer Spaltung. Unter dem N-Terminus bzw. C-Terminus des hh-Proteins, an den bevorzugt gekoppelt wird, sind demnach gemäß der Erfindung die ersten Aminosäuren (N-Terminus) oder die letzten Aminosäuren (C-Terminus) der N- bzw. C-terminalen Domäne zu verstehen. Vorzugsweise wird an eine oder mehrere Aminosäuren der ersten bzw. letzten 10 Aminosäuren gekoppelt. Besonders bevorzugt wird an die erste oder zweite Aminosäure des N-Terminus der N-terminalen Domäne (AS 24 oder 25) bzw. die letzte oder vorletzte Aminosäure des C-Terminus der C-terminalen Domäne (AS 461 oder 462) gekoppelt. Vorzugsweise werden in den erfindungsgemäßen Hedgehog-Konjugaten lipophile Gruppen bzw. die Kohlenwasserstoffkette(n) an die N-terminale Domäne eines hh-Proteins gekoppelt, insbesondere ist das Kopplungsprodukt ein Thioester des N-terminalen Cysteins in Position 24 mit Myristin-, Palmitin-, Palmitolein-, Stearin- oder Olsäure oder ein hh-Protein, das über eine Disulfidbrücke ein Thiocholesterol gebunden hat. Die Herstellung von unmodifiziertem hh-Protein erfolgt vorzugsweise rekombinant mit den dem Fachmann geläufigen Verfahren, vorzugsweise in einem E.coli-Expressionssystem.

Die erfindungsgemäße pharmazeutische Zusammensetzung enthält eine pharmakologisch effektive Dosis des hh-Konjugats und kann lokal appliziert werden. Es ist bevorzugt, die erfindungsgemäßen Konjugate in Kombination mit anderen Proteinen der Hedgehog-Familie oder Knochenwachstumsfaktoren wie bone morphogenetic proteins (BMPs) (Wozney et al., Cell. Mol. Biol. of Bone, Bone Morphogenetic Proteins and their Gene Expression (1993) Academic Press Inc., 131-167) oder Parathyroidhormonen (Karablis et al., Genes and Development 8 (1994) 277-289) oder insulin-like growth factors (IGF-I oder II) oder transforming growth factors (TGF-β) zu verwenden.

In einer weiteren bevorzugten Ausführungsform ist eine pharmazeutische Zusammensetzung des erfindungsgemäßen Hedgehog-Konjugates mit Suramin bevorzugt und diese vorteilhaft verwendbar.

In einer bevorzugten Ausführungsform enthält die pharmazeutische Zusammensetzung Hedgehog-Konjugat in einer Konzentration von 0,01-10 mg/ml, insbesondere 0,01 bis 1 mg/ml.

In einer bevorzugten Ausführungsform enthält die pharmazeutische Zusammensetzung zusätzlich einen pharmazeutisch akzeptablen Puffer, der bioverträglich ist, vorzugsweise im Bereich zwischen pH 4 und pH 10, besonders bevorzugt im Bereich zwischen pH 6 und 9, insbesondere bei einem pH-Wert von ca. pH 7. Der pH-Wert der pharmazeutischen Zusammensetzung sollte zweckmäßig größer pH 4 sein, um eine Denaturierung der gefalteten Struktur und die Ablösung des im Hedgehog-Protein komplexierten Zinks zu verhindern. Die Konzentration des Puffers beträgt vorzugsweise 1-500 mmol/l, vorzugsweise 10-100 mmol/l. In einer geeigneten Ausführungsform wird somit als Puffer 20 mmol/l Kaliumphosphatpuffer, pH 7,2 verwendet.

Zur Herstellung der pharmazeutischen Zusammensetzung ist es weiter bevorzugt, Hilfsstoffe, wie Zucker (Mannitol, Sucrose, Laktose, Glucose, Saccharose, Trehalose, vorzugsweise 20-100 mg/ml) oder Aminosäure, wie Glycin oder Arginin, sowie Antioxidantien, wie EDTA, Citrat, Polyethylenglycol (1-10 Gew.%), Ascorbinsäure, Tocophenol, Detergenzien, vorzugsweise nicht-ionische Detergentien (vorzugsweise 0,005-1 Gew.%), wie Polysorbate (Tween® 20 oder Tween® 80) oder Polyoxyethylene oder auch ionische Detergenzien wie Natriumcholat, -deoxycholat oder -taurodeoxycholat, antiinflammatorische Wirkstoffe, lokale Anästhetika, Antibiotika und/oder Stabilisatoren wie Lipide, Fettsäuren, Glycerin zuzusetzen.

Das erfindungsgemäße Konjugat kann vorteilhaft zur Induktion von Chondrozyten und Osteozyten in einer osteoinduktiven pharmazeutischen Zusammensetzung oder auch zur Induktion von Muskel- und Nervenzellen verwendet werden. Osteoinduktive pharmazeutische Zusammensetzungen sind beispielsweise aus US-Patent 5,364,839, WO 97/35607, WO 95/16035 bekannt.

Bei einer lokalen Applikation des erfindungsgemäßen Konjugats ist bevorzugt, dieses in Kombination mit einer geeigneten Matrix als Träger und/oder einem Sequestierungsagens zu verwenden. Eine solche Matrix ist dazu geeignet, das Protein, insbesondere in der Umgebung von Knochen, in vivo langsam in aktiver Form freizusetzen. Das Sequestierungsagens ist eine Substanz, welche die Applikation, beispielsweise durch Injektion, erleichtert und/oder die Migration des erfindungsgemäßen Proteins von der Applikationsstelle verhindert oder zumindest verzögert.

Vorzugsweise enthält die erfindungsgemäße pharmazeutische Zusammensetzung ein Polymeres (Gerüstsubstanz), das eine Adhäsionsfunktion für Zellen besitzt. Eine solche Gerüstsubstanz ist beispielsweise Collagen.

Als Matrixmaterial ist insbesondere ein biokompatibles degradierbares Material, beispielsweise auf Kollagenbasis oder anderen Polymeren, basierend auf Polymilchsäure, Polyglykolsäure oder Co-Polymeren aus Milchsäure und Glykolsäure geeignet. Solche Polymermatrices sind beispielsweise in der WO 93/00050 beschrieben.

Sequestierungsagentien sind beispielsweise Cellulose und celluloseartige Materialien sowie beispielsweise Alkylcellulose, Carboxymethylcellulose, Hyaluronsäure, Natriumalginat, Polyäthylenglykol und Polyvenylalkohol, wobei Hyaluronsäure, insbesondere in einer pharmazeutischen Zusammensetzung auch ohne Trägermatrix, besonders bevorzugt ist.

Die folgenden Beispiele, Publikationen, das Sequenzprotokoll und die Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beschreibung der Figuren:

- **Figur 1**: zeigt die Aktivität von rekombinantem humanem shh nach Derivatisierung mit Palmitoyl-CoA.
- **Figur 2**: zeigt die Aktivität von rekombinantem humanem shh nach Derivatisierung mit Thiocholesterin.

### Beispiel 1

### Klonierung von humanem sonic hedgehog unter Anhängen eines His-6-Ankers, sowie einer Enterokinase-Schnittstelle; Expression in E. coli

Zur Amplifikation des reifen, N-terminalen Teils von humanem sonic hedgehog (aa 24 - Cys -bis 197 - Gly) aus einem beliebigen Plasmid oder der entsprechenden cDNA, die sonic hedgehog enthalten, kann folgendermaßen vorgegangen werden:

Mit Hilfe zweier Primer (344 und 345) wird ein Stück des shh-Gens von einer internen RsrII Schnittstelle bis zur kodierenden Sequenz für die Aminosäure 198 amplifiziert, wobei gleichzeitig am C-Terminus mehrere Stop-Codons sowie eine PstI-Schnittstelle angehängt werden können.

SEQ ID NO:1

Primer 344: 5' - ca gaattc ttg cggaccg ggc agg gg 26-mer
EcoRI RsrII

SEQ ID NO:2

Primer 345: 5' - ga ctgcag tta a tca tta gcc tcc cga ttt ggc cgc 36-mer
PstI stop stop stop

Ein so amplifiziertes DNA-Fragment kann mit RsrII und PstI nachgeschnitten werden und wird in den folgenden Schritten benötigt (Fragment 344/345).

Durch Annealing zweier weiterer Primer (346 und 347) wird ein Linker hergestellt, mit dessen Hilfe am N-terminus 6 Histidin-Reste sowie eine Enterokinase-Schnittstelle (EK) angebracht werden:

SEQ ID NO:3

Primer 346: 5' - aattc atg cat cat cac cac cac cac gat gac gac gac aaa tg cg
|
His6
Ek
EcoRI Überhang

SEQ ID NO:4

Primer 347: 5' - gtc cgc att tgt cgt cgt cat cgt ggt ggt ggt gat gat gca tg
|
RsrII Überhang

Adapter nach Annealing von 346 und 347:

aattc atg cat cat cac cac cac cac gat gac gac gac aaa tgc g
    g tac gta gta gtg gtg gtg gtg cta ctg ctg ctg ttt acg c ctg

Zur Expression wird nun PCR-Fragment 344/345 zusammen mit Adapter 346/347 in einen mit EcoRI / PstI geschnittenen Vektor kloniert. Dies kann entweder direkt oder nach Zwischerklonierung von Fragment 344/345 in einen anderen Vektor erfolgen. Der EcoRI / PstI geschnittene Vektor muß am EcoRI-Ende einen geeigneten Promotor für Expression in E. coli enthalten, vorzugsweise T5, tac, lac etc. Zur Expression werden die sHH-Expressionsplasmide in einen geeigneten E. coli Stamm transfiziert

### Beispiel 2

### Fermentation

10 l Fermentation des E. coli Expressionsklons für Hedgehog:

Aus Stammkulturen (Plattenausstrich oder bei -20°C gelagerten Ampullen) werden Vorkulturen angesetzt, die geschüttelt bei 30 - 37°C inkubiert werden. Das Überimpfvolumen in die nächsthöhere Dimension beträgt jeweis 1 - 10 Vol%. Zur Selektion gegen Plasmidverlust wird in Vor- und Hauptkultur Ampicillin (50-100 mg/l) eingesetzt.

Als Nährstoffe können enzymatisch verdautes Eiweiß und/oder Hefeextrakt als N- und C-Quelle sowie Glycerin und/oder Glucose als zusätzliche C-Quelle verwendet werden. Das Medium wird auf pH 7 gepuffert und Metallsalze können zur Stabilisierung des Fermentationsprozeßes in physiologisch verträglichen Konzentrationen zugesetzt werden. Die Fermentationstemperatur beträgt 25-37°C. Das Wachstum wird über Ermittlung der optischen Dichte bei 528 nm bestimmt. Mittels IPTG wird die Expression induziert. Nach einer Fermentationsdauer von ca. 30 Std. wird bei OD-Stillstand die Biomasse durch Zentrifugation geerntet.

### Beispiel 3

### Herstellung von unmodifiziertem rekombinantem humanem shh

55 g der in Beispiel 2 hergestellten Biomasse wurden mittels Hochdruckpresse aufgeschlossen, zentrifugiert und der Überstandes auf eine 50 ml Chelating Sepharose (Pharmacia Biotech) aufgetragen, die vorher mit beladen worden war. Die Elution des shh Fusionsproteins erfolgte durch einen Gradienten von 0 bis 200 mM Imidazol in 50 mM Hepes; 250 mM NaCl; pH 7.4. shh enthaltende Fraktionen wurden mittels SDS-PAGE identifiziert, vereinigt und mit einem Volumen 50 mM Hepes; pH 7.4 verdünnt. Bei der Verdünnung auftretende Präzipitate wurden abzentrifugiert und der Überstand gegen 50 mM Hepes; pH 7.4 bei 4 ° C dialysiert. 500 mg des so erhaltenen shh Fusionsproteins wurden mit Enterokinase (1:500; ww; Boehringer Mannheim GmbH) und β-ME (ad 10 mM) versetzt und 16 h bei 35°C im Wasserbad inkubiert. Anschließend wurde festes DTT zu einer Konzentration von 10 mM zugegeben. Die Probe wurde auf eine 166 ml SP-Sepharose (Pharmacia Biotech) auftragen und im Gradienten von 0-800 mM NaCl in 20 mM HEPES, pH 7.4 eluiert. Nach Analyse der Peakfraktionen mittels SDS-PAGE wurden die Hauptfraktionen vereinigt, aliquotiert und bei -80°C bis zur weiteren Verwendung gelagert. Diese Hauptfraktionen enthalten shh unter nicht-reduzierenden Bedingungen als Dimer, das über eine reduzierbare Disulfidbrücke verknüpft ist und ein apparentes Molekulargewicht von ca. 38 kDa aufweist.

### Beispiel 4

### Herstellung von palmitoyliertem rekombinantem humanem shh mit Palmitoyl-CoA:

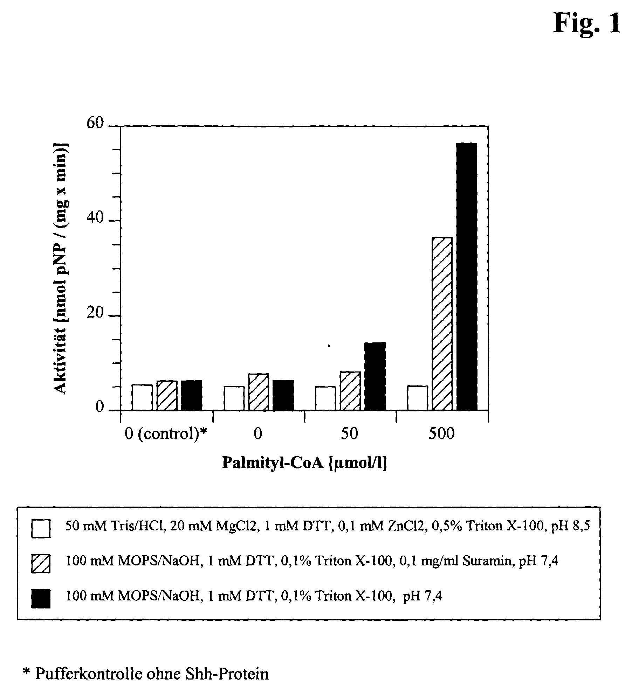

Je 2 ml der gereinigten shh Probe mit einer shh Konzentration von 0,35 mg/ml wurden mit DTE ad 20 mM versetzt und 2 h bei 37°C inkubiert und anschließend gegen
a) 50 mM Tris/HCl, 1 mM DTE, 0,1 mM ZnCl2, 0,5 % Triton® X-100, pH 8,5
b) 100 mM MOPS, 1 mM DTE, 0,1 % Triton® X-100, 0, 1 mg/ml Suramin, pH 7,4
c) 100 mM MOPS, 1 mM DTE, 0,1 % Triton® X-100, pH 7,4
dialysiert. Anschließend wurden zu je 0,5 ml der Proben unterschiedliche Volumina einer Palmitoyl-CoA-Lösung (10 mg/ml in 100 mM MOPS, 1 mM DTT, 0,2% Triton® X-100, pH 7,6) zugegeben, die in den Ansätzen Palmitoyl-CoA-Konzentrationen von
1) 0 µM
2) 50 µM
3) 500 µM
ergaben, worauf 1 h bei 37°C inkubiert wurde. Vor der Filtration und 1/200-Verdünnung für den Zelltest wurden die Proben dann mit BSA ad 1 mg/ml und Suramin ad 0,1 mg/ml versetzt. Wie der in Beispiel 5 beschrieben Aktivitätstest für shh mit obigen Proben ergab, zeigen die in Puffern b) und c) mit 500 µM Palmitoyl-CoA inkubierten shh Proben eine drastisch erhöhte biologische Aktivität. (siehe Fig. 1) im Vergleich zu den Ansätzen ohne Palmitoyl-CoA. Eine Reinigung des derart palmitoylierten shh ist mit Methoden möglich, wie sie für Membranproteine z.B. in 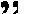 A practical Guide to Membrane Protein Purification" (1994; ed.: Jagow & Schlägger; Academic Press) oder in der Europäischen Patentanmeldung Nr. 98 102 095.1 beschrieben sind.

### Beispiel 5

### Derivatisierung von rekombinantem humanem shh mit Thiocholesterin

Zur Modifikation von shh durch ein am aminoterminalen Cystein über eine Disulfidbrücke verknüpftes Thiocholesterin wurde reduziertes, monomers shh mit einer Konzentration von 0,66 mg/ml in 0,5 mM DTT pH 7,0 im Verhältnis 1:3 in folgende Reaktionspuffer verdünnt:
100 mM Ethanolamin
100 mM NaCl
50 µM CuCl₂
pH 9,5
0,8% (w/v) Natriumdesoxycholat oder 0,4% (w/v) Natriumcholat oder 1,3% (w/v) n-Octylglykosid oder 0,3% Triton® X-100.
   Zum Start der Reaktion wurden ad
5% (v/v) Aceton oder 100 µM Thiocholesterin (aus einer 10 mM Lösung in Aceton) oder 500 µM Thiocholesterin (aus einer 10 mM Lösung in Aceton)
zugesetzt und 30 min. bei Raumtemperatur geschüttelt.

Die Proben wurden bereits vor der Sterilfiltration mit dem neunfachen Volumen 20 mM Natriumphosphat, 0,9% NaCl, 0,05% Tween® 80, 1 mg/ml, 0,1 mg/ml Suramin, pH 7,2 verdünnt und in einer weiteren 1/20-Verdünnung im Zelltest analysiert.

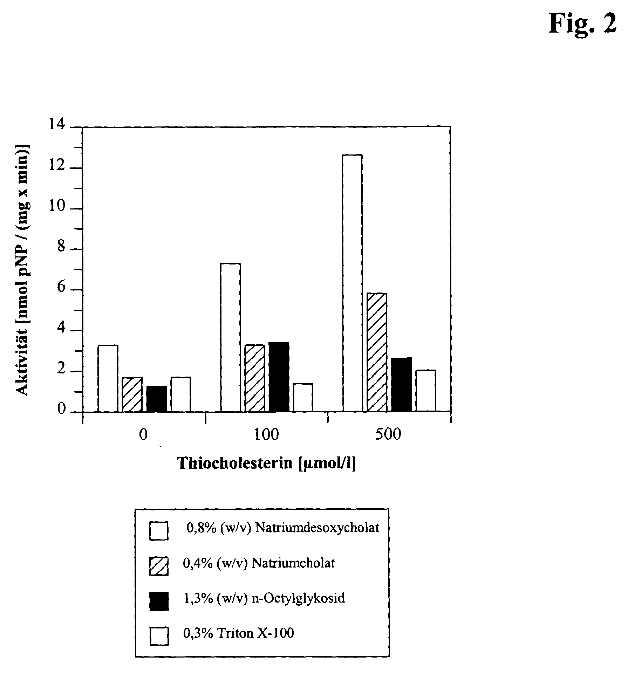

Wie in Fig. 2 dargestellt, zeigte sich in Abhängigkeit von der Thiocholesterinkonzentration eine Zunahme der Aktivität, die in den Proben mit den anionischen Detergenzien am effektivsten generiert bzw. stabilisiert wurde.

### Beispiel 6

### Induktion von alkalischer Phosphatase im Zelltest (Bestimmung der Aktivität der Alkalischen Phosphatase)

5000 Zellen der murinen mesenchymalen pluripotenten Linie C3H10T1/2 (ATCC CCL-226) wurden pro Vertiefung einer 96-Loch Mikrotiterplatte eingesät. Die Zellen befanden sich in 100 µl DMEM, 2mM Glutamin, 100 IU/ml Penicillin, 100 ug/ml Streptomycin und 10 % fötalem Kälberserum, FKS. Am nächsten Tag wurden die zu untersuchenden Wirkstoffe in den entsprechenden Konzentrationen nach Verdünnung in Kulturmedium in einem Volumen von 100 µl zugefügt. Nach 5 Tagen wurde der Test gestoppt. Dazu wurden die Überstände abgekippt und die Zellen einmal mit PBS gewaschen. Die Zellen wurden lysiert in 50 µl 0.1 % Triton® X-100 und bei -20 C eingefroren. Nach dem Auftauen wurden 25 µl für die Proteinbestimmung und 25 µl für die Bestimmung der Aktivität der Alkalischen Phosphatase eingesetzt.

### Proteinbestimmung nach der Anleitung des Herstellers Pierce :

Der Ansatz wurde mit 75 µl H2O bidest. versetzt, dann wurden 100 µl BCA Protein Reagenz zugefügt (Pierce Micro BCA, Nr. 23225). Nach 60 min wurde die Optische Dichte (O.D.) bei 550 nm gemessen.

### Aktivität der Alkalischen Phosphatase nach der Anleitung des Herstellers Sigma :

Der Ansatz wurde mit 100 µl Reaktionspuffer (Sigma 221) versetzt. Eine Substratkapsel (Sigma 104-40) wurde in 10 ml H₂O bidest. aufgelöst, dann wurden 100 µl zu dem Testansatz pipettiert. Die O.D. wurde nach der Gelbfärbung bei 405 nm gemessen. Bei der Reaktion setzt die Alkalische Phosphatase p-Nitrophenyl-phosphat zu p-Nitrophenol um.

Die O.D. wurden jeweils mittels Standardkurven in nmol bzw. µg umgerechnet. Die Auswertung erfolgte nach der Formel :
*nmol PNP pro (Meß) Minute pro mg (Zell) Protein*

### Beispiel 7

### In vivo Aktivität von palmitoyliertem shh

Die in vivo Aktivität von modifizierten (Beispiel 4) und unmodifizierten shh Protein (Beispiel 3) (hergestellt durch cytoplasmatische Expression in E.coli) wurde in einem für Knochenwachstumsfaktoren etablierten Tiermodell untersucht (Mackie & Trechsel (1990) Bone 11, 296; L. Kling et al. (1996) J.Bone Min.Res. 11 (Suppl.1), S153).

Sieben Wochen alten weiblichen BALB/c Mäusen wurden über einen Zeitraum von 15 Tagen täglich 1, 10 oder 50 ug shh in einem Volumen von 50 ul subkutan an die Schädelkalotte injiziert. 14 Tage nach Beendigung der Behandlung wurden die Kalotten entnommen und von umgebenden Bindegewebe gereinigt. Anschließend wurden die Gewichte der normierten Explantate und die Röntgendichte analysiert.

Nach Beispiel 3 modifizierter shh zeigte im Vergleich zu unmodifizierten shh eine höhere osteoanabole Wirkung.

### Referenzliste

A Practical guide to membrane protein purification, Ed. G. v. Jagow, Hermann Schägger (1994), Kapitel 16, Seiten 535 - 554
Asahina, J., Exp. Cell. Res. 222 (1996) 38 - 47
Bitgood, M.J. et al., Curr. Biol. 6 (1996) 296
Bizzozero et al., J. Biol. Chem. 262 (1987) 2138 - 2145
Chiang, C. et al., Nature 83 (1996) 407
Europäische Patentanmeldung Nr. 98 102 095.1
Fietz, M. et al., Development (Suppl.) (1994) 43 - 51
Gaertner et al., Bioconjugate Chem. 3 (1992) 262-268
Hancock, J.F., Cell 63 (1990) 133 - 139
Haque, Z. et al., Agric. Biol. Chem. 46 (1982) 597 - 599
Haque, Z. et al., J. Agric. Food Chem. 30 (1982) 481
Haque, Z. et al., J. Agric. Food Chem. 31 (1983) 1225 - 1230
Hynes, M. et al., Neuron 15 (1995) 35 - 44
Karablis et al., Genes and Development 8 (1994) 277 - 289
Kinto et al., FEBS Letters, 404 (1997) 319 - 323
Kling, L. et al. (1996) J.Bone Min.Res. 11 (Suppl.1), S153
Lai, C.J. et al., Development 121 (1995) 2349
Liu et al., Proc. Natl. Acad. Sci. USA 91 (1994) 6584-6588
Lopez-Martinez et al. in Curr. Biol. 5 (1995) 791-796
Lundblad, Techniques in Protein Modification, CRC Press, Boca Raton, FL, USA (1995)
Mackie & Trechsel (1990) Bone 11, 296
Marti et al., Nature 375 (1995) 322-325
Nakamura, T. et al., Biochem. Biophys. Res. Comm. 237 (1997) 465 - 469
Ozols, J. et al., Molec. Biol. of the Cell 8 (1997) 637 - 645
Perrimon, N., Cell 80 (1995) 517 - 520
Porter, J.A. et al., Science 274 (1996) 255 - 259
Ross et al., J. Neurosci. Res. 21 (1988) 35 - 44
Segawa, A. et al., Int. Archs Allergy appl. Immun. 66 (1981) 189 - 199
Skolnick et al., Biol. Membranes (1996) 536 - 554; ed.: Merz and Roux
Smith, J.C., Cell 76 (1994) 193 - 196
US-Patent 5,364,839
Vortkamp, A. et al., Science 273 (1996) 613
Webb, R.J. et al., Biochemistry 37 (1998) 673 - 679
WO 93/00050
WO 95/16035
WO 97/35607
Wong, S.S.,Chemistry of Protein Conjugation and Cross Linking, CRC Press, Boca Raton, USA, 1993
Wozney et al., Cell. Mol. Biol. of Bone, Bone Morphogenetic Proteins and their Gene Expression (1993), Academic Press Inc., 131 - 167
Yadav, S.P. et al., Biochem. Biophys. Res. Comm. 205 (1994) 1688 - 1695
Yang et al., Development 124 (1997) 4393-4404

## Patentansprüche

1. Hedgehog-Konjugat, dadurch gekennzeichnet, daß es an einem Hedgehog-Protein kovalent gebunden enthält:
a) ein Polypeptid bestehend aus 10 bis 30 hydrophoben Aminosäuren und/oder aus Aminosäuren, welche Transmembranhelices bilden und positiv geladen sind,
b) 1 bis 4 aliphatische, gesättigte oder ungesättigte Kohlenwasserstoffreste mit einer Kettenlänge von 10 bis 24 C-Atomen und mit einer hydrophoben Wirkung, oder
c) eine hydrophobe Thioverbindung.

2. Hedgehog-Konjugat nach Anspruch 1, dadurch gekennzeichnet, daß das Polypeptid 2 bis 12 Lysine und/oder Arginine enthält.

3. Hedgehog-Konjugat nach Anspruch 1, dadurch gekennzeichnet, daß der Kohlenwasserstoffrest ein Fettsäure- oder Alkylalkoholrest ist, der als Ester, Thioester oder Säureamid gebunden ist.

4. Hedgehog-Konjugat nach Anspruch 3, dadurch gekennzeichnet, daß die Fettsäure Laurinsäure, Myristinsäure, Palmitinsäure, Stearinsäure, Arachinsäure, Behensäure. Palmitoleinsäure, Ölsäure, Linolsäure, Linolensäure oder Arachidonsäure ist.

5. Hedgehog-Konjugat nach Anspruch 1, dadurch gekennzeichnet, daß die hydrophobe Thioverbindung Thiocholesterol ist.

6. Hedgehog-Konjugat nach den Ansprüchen 1 bis 5, dadurch gekennzeichnet, daß der Kohlenwasserstoffrest, das Polypeptid oder die Thioverbindung am C- und/oder N-Terminus des Hedgehog-Proteins gebunden ist.

7. Verfahren zur Herstellung eines Hedgehog-Konjugats nach den Ansprüchen 3 oder 4 und 6, dadurch gekennzeichnet, daß der Kohlenwasserstoffrest kovalent an eine freie Hydroxy-, Mercapto-, Carboxy- oder Aminogruppe des Hedgehog-Proteins gekoppelt wird.

8. Verfahren zur Herstellung eines Hedgehog-Konjugats nach den Ansprüchen 1 bis 6 durch kovalente Kopplung eine Kohlenwasserstoffrestes oder einer hydrophoben Thioverbindung an ein Hedgehog-Protein, dadurch gekennzeichnet, daß die kovalente Kopplung des Kohlenwasserstoffrests des Polypeptids oder der hydrophoben Thioverbindung und/oder die Isolierung in Gegenwart von Suramin, Heparin oder anionischen Polysacchariden erfolgt.

9. Verfahren zur Herstellung eines Hedgehog-Konjugats nach den Ansprüchen 1 bis 6 durch kovalente Kopplung eines Fettsäurerests oder einer hydrophoben Thioverbindung an ein Hedgehog-Protein, dadurch gekennzeichnet, daß als Fettsäurerest Palmitoyl-CoA und als hydrophobe Thioverbindung Thiocholesterol verwendet wird.

10. Pharmazeutische Zusammensetzung enthaltend ein Hedgehog-Konjugat nach den Ansprüchen 1 bis 6 in einer pharmazeutisch wirksamen Menge sowie pharmazeutische Hilfsstoffe, Detergenzien, Stabilisatoren, Matrixmaterialien, Suramin, Heparin, anionische Polysaccharide und/oder Sequestierungsagenzien.

11. Verfahren zur Herstellung einer pharmazeutischen Zusammensetzung, dadurch gekennzeichnet, daß als wesentlicher Bestandteil dieser Zusammensetzung ein Hedgehog-Konjugat nach den Ansprüchen 1 bis 6 verwendet wird.


SEQUENZPROTOKOLL

(1) ALLGEMEINE ANGABEN:

(i) ANMELDER:
(A) NAME: BOEHRINGER MANNHEIM GMBH
(B) STRASSE: Sandhofer Str. 116
(C) ORT: Mannheim
(E) LAND: Germany
(F) POSTLEITZAHL: D-68305
(G) TELEFON: 08856/60-3446
(H) TELEFAX: 08856/60-3451

(ii) BEZEICHNUNG DER ERFINDUNG: Aktives Hedgehog-Protein-Konjugat,
Verfahren zur Herstellung und Verwendung

(iii) ANZAHL DER SEQUENZEN: 4

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30B (EPA)

(2) ANGABEN ZU SEQ ID NO: 1:

(i) SEQUENZKENNZEICHEN:
(A) LÄNGE: 26 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer 344"

(xi) SEQUENZBESCHREIBUNG: SEQ ID NO: 1:

CAGAATTCTT GCGGACCGGG CAGGGG 26

(2) ANGABEN ZU SEQ ID NO: 2:

(i) SEQUENZKENNZEICHEN:
(A) LÄNGE: 36 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer 345"

(xi) SEQUENZBESCHREIBUNG: SEQ ID NO: 2:

GACTGCAGTT AATCATTAGC CTCCCGATTT GGCCGC 36

(2) ANGABEN ZU SEQ ID NO: 3:

(i) SEQUENZKENNZEICHEN:
(A) LÄNGE: 45 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer 346"

(xi) SEQUENZBESCHREIBUNG: SEQ ID NO: 3:

AATTCATGCA TCATCACCAC CACCACGATG ACGACGACAA ATGCG 45

(2) ANGABEN ZU SEQ ID NO: 4:

(i) SEQUENZKENNZEICHEN:
(A) LÄNGE: 44 Basenpaare
(B) ART: Nucleotid
(C) STRANGFORM: Einzelstrang
(D) TOPOLOGIE: linear

(ii) ART DES MOLEKÜLS: other nucleic acid
(A) BESCHREIBUNG: /desc = "Primer 347"

(xi) SEQUENZBESCHREIBUNG: SEQ ID NO: 4:

GTCCGCATTT GTCGTCGTCA TCGTGGTGGT GGTGATGATG CATG 44